# DEMANDE DE BREVET EUROPEEN

(11) **EP 1 733 701 A2**
(43) Date de publication de la demande: **20.12.2006**
(21) Numéro de dépôt: 06018981.8
(22) Date de dépôt: 03.07.2001
(51) Int. Cl.: A61F 2/00, A61B 17/02

(54) **Dispositif implantable destiné à corriger l'incontinence urinaire**

(30) Priorité: 05.07.2000 FR 0008706
(62) Demande divisionnaire de: 01949616.5
(71) Demandeur: Coloplast A/S, DK-3050 Humlebaek (DK)
(72) Inventeur: Suslian, Patrice, 84220 Gordes (FR); Delorme, Emmanuel, 71100 Chalon sur Saone (FR)
(74) Mandataire: Grund, Martin

(57) **Abrégé**

L'invention concerne un dispositif implantable destiné à corriger l'incontinence urinaire chez la femme, caractérisé en ce qu'il se présente sous forme d'une bandelette (1), dont la région centrale (2) destinée à être intercalée entre le facia de Alban et les facias périurétraux est enduite d'une substance apte à empêcher toute adhésion desdits facias sur la bandelette (1).

## Description

L'invention concerne une méthode pour le traitement de l'incontinence urinaire chez la femme. Elle concerne également un dispositif implantable destiné à corriger l'incontinence urinaire chez la femme. Ledit dispositif est plus particulièrement adapté pour le traitement de l'incontinence urinaire d'effort.

Divers types de dispositifs ont été proposés pour traiter les phénomènes d'incontinence urinaire chez la femme.

Ainsi, par exemple, le document US-A-5 899 909 décrit une bandelette de largeur constante, réalisée en une matière du type polypropylène maillé ou tricoté permettant d'assurer sur toute sa longueur la colonisation fibroblastique et par conséquent son ancrage dans les tissus. Cette bandelette est positionnée après incision de la paroi vaginale, sous l'urètre, la bandelette remontant de part et d'autre de la vessie pour venir s'ancrer dans la paroi abdominale.

La méthode de mise en place de cette bandelette est relativement délicate. En effet, les aiguilles en remontant verticalement le long de la vessie peuvent non seulement percer celle-ci, mais surtout percer l'artère iliaque ou encore l'intestin grêle. En conséquence, il est indispensable de pratiquer une cystoscopie pendant l'intervention.

Le document WO 98/35632 décrit un dispositif sous forme d'une bandelette, dont la partie centrale est plus large que le corps de la bandelette, l'ensemble étant réalisé en un matériau biocompatible, notamment un matériau tissé, permettant la colonisation fibroblastique.

De même que précédemment, chacune des extrémités de la bandelette remonte le long de la vessie pour être fixée au niveau de la paroi abdominale ou plus spécifiquement dans l'os du pubis. Dès lors, les mêmes inconvénients que précédemment peuvent être relevés.

L'un des objets de l'invention est la reconstruction artificielle du facia pelvien par la mise en place de bandelettes visant à restituer le plus fidèlement possible la situation efficace et naturelle du facia endo-pelvien, dans son rôle de bouchon fibreux venant obturer la fente uro-génitale, ledit facia reposant de part et d'autre de ladite fente sur le plancher des muscles releveurs.

Un autre des buts visés par la présente invention réside dans la solution aux problèmes liés à la réintervention chirurgicale ultérieure au niveau de l'urètre. En effet, compte tenu du fait que les bandelettes proposées par l'Art Antérieur sont réalisées sur toute leur longueur en un matériau apte à être colonisé par les fibroblastes, le problème se pose d'une intervention dans cette zone si la bandelette, du fait de la colonisation fibroblastique, est ancrée dans la paroi périurétrale. La résolution de ce nouveau problème est d'autant plus importante que l'on constate que le phénomène d'incontinence urinaire peut évoluer vers la mise en place d'un sphincter artificiel. Un tel problème n'est ni divulgué ni suggéré par l'Art Antérieur.

En outre, on a décrit dans la littérature des phénomènes possibles de migration de la matière constitutive de la bandelette et notamment de polypropylène dans les viscères.

Pour résoudre l'ensemble de ces problèmes, le Demandeur propose une méthode et un dispositif implantable, destinées à corriger l'incontinence urinaire chez la femme.

Cette méthode de traitement de l'incontinence urinaire chez la femme comprend les étapes suivantes :
• réalisation d'une incision paraurétrale médiane sensiblement au niveau du tiers moyen de l'urètre mesuré à partir du méat de telle sorte à permettre le passage d'une bandelette entre le facia de Alban et les facias périurétraux ;
• extension de chacune des extrémités libres de ladite bandelette au niveau des deux trous obturateurs de l'aile iliaque et sortie dans l'aine en regard du trou correspondant de telle sorte à former sensiblement un V, dont la pointe passe sous l'urètre sans en modifier la position.

En d'autres termes et contrairement au techniques chirurgicales mises en oeuvre dans l'Art Antérieur, la bandelette ne remonte pas le long de la vessie pour former un U, et ainsi se retrouver dans le voisinage immédiat d'organes vitaux, mais au contraire s'en écarte pour former un V. Dès lors, aucun risque d'endommagement de 1a vessie, de l'artère iliaque ou encore de l'intestin grêle n'est encouru. En conséquence, il n'est pas nécessaire de procéder à une cystoscopie pendant l'intervention.

Selon l'invention, afin de favoriser la mise en place de la bandelette faisant fonction d'implant, on définit un espace entre d'une part le facia de Alban, le plan périnéal musculaire et l'insertion antérieure du muscle puborectal, et d'autre part les facias périurétraux.

Selon une version avantageuse de l'invention, on enduit la région centrale de la bandelette ou implant, destinée à être intercalée entre la facia de Alban et les facias périutréraux d'une substance aptre à empêcher toute adhésion desdits facias sur la bandelette.

De la sorte, on s'affranchit de toutes proliférations cellulaires sur la bandelette entre 1a paroi vaginale et la paroi urétrale, évitant ainsi tout ancrage de la bandelette dans cette région, pour ainsi permettre une réintervention chirurgicale ultérieure. En outre, l'enduction d'une telle substance au niveau de l'urètre permet d'éviter toute migration de polypropylène dans les viscères.

Le dispositif conforme à l'invention se caractérise en ce qu'il se présente sous forme d'une bandelette, dont la région centrale destinée à être intercalée entre le facia de Alban et le facia périurétral est enduite d'une substance apte à empêcher toute adhésion desdits facias sur la bandelette.

Dans une première forme de réalisation, la substance empêchant l'adhésion des facias sur la bandelette est de la silicone.

Dans une seconde forme de réalisation, la substance est constituée de facteurs de croissance d'origine végétale ou animale.

Bien entendu, toute substance apte à éviter l'adhésion des facias sur la bandelette peut être envisagée.

L'enduction de la bandelette par ladite substance peut être réalisée sur les deux faces, avantageusement une face.

Par ailleurs, la bandelette est fabriquée en tous matériaux tels que ceux connus de l'homme du métier et en particulier mais de façon non limitative, tous matériaux choisis dans le groupe comprenant le polyéthylène et le polypropylène.

Selon une autre caractéristique, lorsque la bandelette est réalisée en polypropylène, le polypropylène est soit maillé, soit tricoté, ou encore sous forme de fibres projetées. Cependant, la bandelette peut également être fabriquée en un matériau résorbable.

Dans une forme de réalisation avantageuse, la région centrale de la bandelette est de moindre largeur par rapport au reste de la bandelette et ce, afin de limiter la surface de contact au niveau du facia de Alban et du facia périurétral.

Par ailleurs et selon une autre caractéristique, chacune des extrémités de la bandelette présente une pointe effilée destinée à être ancrée dans l'aine en regard du trou obturateur correspondant.

Selon une forme de réalisation avantageuse, la bandelette a une longueur égale à 60 centimètres et une largeur égale à 2,5 centimètres, et présente une région centrale de largeur moindre, égale à 1 centimètre sur une longueur égale à 3 centimètres. Préférentiellement, la longueur de la région centrale est égale à 15 mm.

L'invention et les avantages qui en découlent ressortiront mieux de l'exemple de réalisation suivant à l'appui des figures annexées.

La figure 1 est une représentation schématique de la bandelette de l'invention.

La figure 2 est une représentation schématique du positionnement de la bandelette après mise en place.

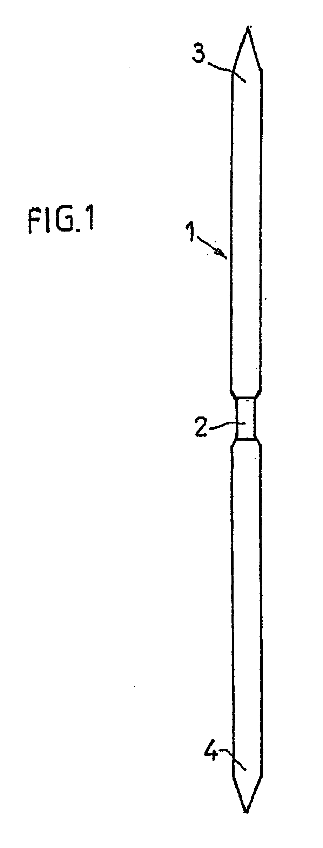

Comme le montre la figure 1, le dispositif de l'invention se présente sous forme d'une bandelette (1) de longueur égale à 60 centimètres et de largeur égale à 2,5 centimètres. Cette bandelette est réalisée, sur toute sa longueur, en fibres projetées de polypropylène.

Selon une caractéristique avantageuse, la bandelette présente en son centre (2) un rétrécissement de longueur égale à 3 centimètres et de largeur égale à 1 centimètre, cette portion étant enduite sur ses deux faces d'une matière siliconée. En outre, chacune de ses extrémités libres (3, 4) est effilée.

La mise en place du dispositif nécessite une chirurgie mini-invasive, dont les principales étapes sont décrites ci-après.

On procède tout d'abord à une incision paraurétrale médiane au niveau du tiers moyen de l'urètre.

On repère ensuite l'un des deux trous obturateurs, et plus précisément la partie inférointerne au moyen d'un doigt glissé dans l'incision vaginale et on incise la peau périnéale en regard de celui-ci, donc dans l'aine, de sorte à ménager un orifice par lequel on introduit ensuite une aiguille de Emmet. Celle-ci est introduite par cette incision cutanée d'abord perpendiculairement au périnée sur environ 15 mm, (traversée du muscle obturateur interne juste en dehors de la branche ischipubienne), puis on laisse l'aiguille décrire sa courbe, guidée en cela par le doigt introduit en regard du muscle obturateur par l'incision vaginale. On passe alors l'extrémité en pointe de la bandelette dans le chas de l'aiguille, ressortant par ladite incision vaginale , puis on tracte par retour à travers l'épaisseur musculaire, le releveur et l'obturateur interne, jusqu'à la surface de la peau.

On place ensuite la bandelette entre le facia de Alban et les facias périurétraux pour la positionner de sorte à ce que sa zone centrale, le cas échéant enduite de silicone se trouve en regard desdits facias. La bandelette est posée sans traction derrière l'urètre.

On procède alors à une incision de la peau périnéale en regard du second trou obturateur, dans lequel on introduit une aiguille de EMMET. On passe alors l'extrémité libre de la bandelette dans le chas de l'aiguille que l'on tracte par retour de la même manière que précédemment.

On sectionne ensuite la bandelette excédentaire au ras de la peau puis on immobilise la peau de façon à la désolidariser de la bandelette. On ferme enfin par un point de fil rapidement résorbable.

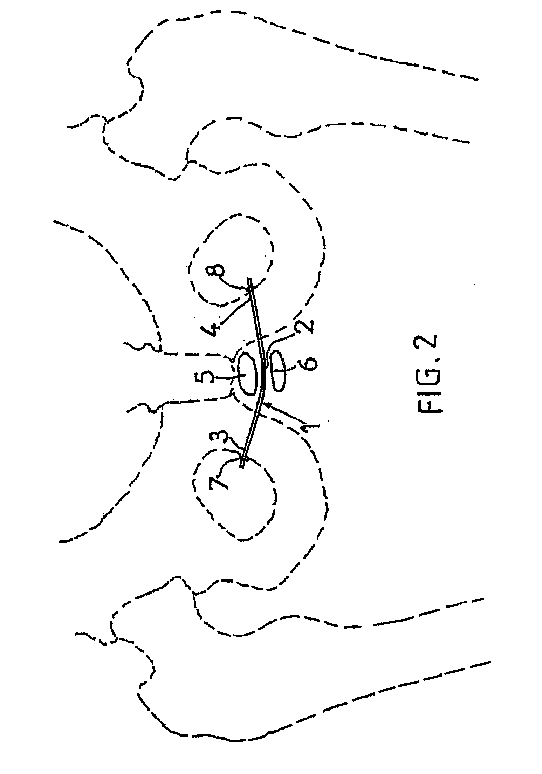

Sur la figure 2, on a représenté schématiquement le positionnement en coupe de la bandelette après sa mise en place. Comme le montre cette figure, une fois en place, la bandelette adopte une forme en V, dont les branches sont très écartées.

En outre, on peut également observer, que lorsque l'on met en oeuvre la bandelette conforme à l'une des formes avantageuses de l'invention, le segment siliconé de la bandelette est positionné entre l'utérus (5) et le vagin (6), tandis que ses extrémités (3, 4) sont fixées au niveau de l'aine (7, 8) en regard du trou obturateur.

Grâce à la solidité et à 1a texture de la bandelette, la traction peut être forte sans qu'il n'y ait aucun risque de rupture. La bandelette est positionnée sous contrôle de la vue sans mise en oeuvre de cystoscopie. Il est essentiel qu'il n'y ait aucune traction sur cette bandelette qui doit être déposée sous l'urètre sans en modifier la position.

Il ressort de ce qui précède que la méthode de traitement de l'incontinence urinaire chez la femme conforme à l'invention se distingue ce celles proposées dans l'état de la technique par la simplicité de mise en place de la bandelette, en mettant en oeuvre une chirurgie mini-invasive. En outre, elle assure une suspension urétrale solide tout en gardant une certaine souplesse, et surtout en maintenant relativement éloignée de ladite bandelette les organes vitaux du voisinage. Par ailleurs, elle participe à la reconstruction du facia endopelvien.

## Revendications

1. Dispositif implantable pour corriger l'incontinence urinaire chez les femmes, **caractérisé en ce qu'**il présente la forme d'une bandelette avec une région centrale conçue pour être insérée entre le fascia de Halban et le fascia périurétral, et qu'une partie de ladite bandelette est adaptée pour s'étendre depuis le dessous de l'urètre à travers un foramen obturateur et qu'une autre partie de ladite bandelette est adaptée pour s'étendre depuis le dessous de l'urètre à travers l'autre foramen obturateur.

2. Dispositif implantable selon la revendication 1, dans lequel la bandelette est adaptée pour former essentiellement un V une fois en place.

3. Dispositif implantable selon la revendication 1 ou 2, dans lequel la région centrale de la bandelette n'est pas aussi large que le reste de la bandelette afin de limiter la surface de contact dans la région du fascia de Halban et du fascia périurétral.

4. Dispositif implantable selon la revendication 1, 2 ou 3, dans lequel chacune des extrémités de la bandelette présente une pointe effilée destinée à être fixée dans l'aine en regard du foramen obturateur correspondant.

5. Dispositif implantable selon l'une quelconque des revendications 1 à 4, dans lequel la région centrale de la bandelette, qui est conçue pour être insérée entre le fascia de Halban et le fascia périurétral, est revêtue d'une substance capable d'empêcher toute adhérence desdits fasciae à la bandelette.

6. Dispositif implantable selon la revendication 5, dans lequel ladite substance capable d'empêcher l'adhérence des fasciae à la bandelette est du silicone.

7. Dispositif implantable selon la revendication 5, dans lequel la substance capable d'empêcher l'adhérence des fasciae à la bandelette est composée de facteurs de croissance végétaux ou animaux.

8. Dispositif implantable selon l'une quelconque des revendications 1 à 7, dans lequel la bandelette a une longueur de 60 centimètres et une largeur de 2,5 centimètres.

9. Dispositif implantable selon l'une quelconque des revendications 1 à 8, dans lequel la région centrale est plus étroite que le reste de la bandelette.

10. Dispositif implantable selon la revendication 9, dans lequel la largeur de la région centrale est de 1 centimètre pour une longueur de 3 centimètres, de préférence pour une longueur de 1,5 millimètres.

11. Dispositif selon l'une quelconque des revendications 1 à 10, dans lequel ladite bandelette est faite en un matériau choisi dans le groupe comprenant le polyéthylène et le polypropylène.

12. Dispositif implantable selon l'une quelconque des revendications 1 à 10, dans lequel ladite bandelette est faite en polypropylène et est soit un matériau maillé ou tissé, soit sous la forme de fibres projetées.
